# EUROPÄISCHE PATENTSCHRIFT

(11) **EP 1 043 582 B1**
(45) Veröffentlichungstag und Bekanntmachung des Hinweises auf die Patenterteilung: **18.08.2004**
(21) Anmeldenummer: 00105379.2
(22) Anmeldetag: 21.03.2000
(51) Int. Cl.: G01N 21/76

(54) **Diffusionskontrollierende Sensorschicht**
Diffusion-controlling sensor layer
Couche sensible régulatrice de diffusion

(30) Priorität: 03.04.1999 DE 19915310
(43) Veröffentlichungstag der Anmeldung: 11.10.2000
(60) Teilanmeldung: 02014011.7
(73) Patentinhaber: BAYER AG, 51368 Leverkusen (DE)
(72) Erfinder: Kreiss, Wolfgang, Dr., 51467 Bergisch Gladbach (DE); Eberz, Günther, Dr., 51519 Odenthal-Holz (DE); Weisemann, Claus, Dr., Apex, NC 27502 (US); Burger, Klaus, Dr., 41468 Neuss (DE)

(56) Entgegenhaltungen:
- US-A- 4 162 208
- US-A- 5 578 460
- US-A- 5 766 963
- P.D. SHAW ET AL.: "Detecting and characterizing N-acyl-homoserine lactone signal molecules by thin-layer chromatography" PROCEEDINGS OF THE NATIONAL ACADEMY OF SCIENCES OF THE UNITED STATES OF AMERICA, Bd. 94, Nr. 12, 1997, Seiten 6036-6041, XP000990381 NATIONAL ACADEMY OF SCIENCE, WASHINGTON, DC, US ISSN: 0027-8424

## Beschreibung

Aussagen zur biologischen Wirkung von Substanzen sind wesentlich für die Entwicklung und Anwendung von Wirkstoffen, insbesondere im pharmazeutischen Bereich. Wirkungsprüfungen stellen entscheidende Schritte bei der Bewertung der Ergebnisse der Kombinatorischen Chemie sowie der Evaluierung von Naturstoffen oder von synthetischen Substanzbibliotheken dar. Die Erfindung betrifft eine diffusionskontrollierende Sensorschicht, eine Vorrichtung und ein Verfahren zur Erfassung der biologischen Wirkung von Substanzen.

Zur Prüfung von biologischen Wirkungen werden im allgemeinen Mikrotiterplatten-Formate oder davon abgeleitete Formate angewendet. Gemeinsames Merkmal dieser Techniken ist die Durchführung des Wirkungsassays in abgeschlossenen Kompartimenten (Mikrotiterplatten-Well, Vial, Sensorpunkt in Sensorarray). Dabei wird die zu prüfende Substanz in diskreten Flüssigkeitsvolumina, z. B. innerhalb der Mikrotiterplatten-Wells, mit dem Sensorsystem in Kontakt gebracht. Biologische Wirkung wird in den einzelnen Mikrotiterplatten-Wells über die jeweilige Reaktion des Sensorsystems, z. B. Farbänderung in Gegenwart bioaktiver Substanzen, erkannt [High Throughput Screening, John P. Devlin, Marcel Dekker INC, New York, 1997]. Nach dem Stand der Technik befinden sich die Sensororganismen in einer Suspension, in der sie frei diffundieren können. In einer Suspension kann es zu einer Sedimentation der Sensororganismen während des Detektionsprozesses kommen. Außerdem wird keine gleichmäßige Beschichtung unterschiedlicher Materialien (Glas, Kunststoff, Metall) und Oberflächen (glatt, rauh, porös) erreicht.

Die dem Stand der Technik entsprechenden Prüfverfahren ergeben zwar Wirkungsaussagen zu einer Probe als Ganzes, sie sind aber aufgrund ihrer diskontinuierlichen Arbeitsweise nicht in der Lage, die örtliche Verteilung von biologischer Aktivität auf der Oberfläche von Untersuchungsobjekten abzubilden.

Eine weitere Einschränkung für Testverfahren nach dem Stand der Technik besteht in der Empfindlichkeit gegenüber Störungen, die zu falsch negativen Aussagen führen. So werden konventionelle Zell-basierte Tests durch cytotoxische Substanzen gestört, während enzymatische Tests z. B. durch denaturierende Substanzen beeinflusst werden. Diese störenden Komponenten können als Kontamination in der zu prüfenden Probe vorliegen oder sind Bestandteil des Substanzgemischs, wie es bei Naturstoffen oft der Fall ist.

Bei den etablierten Wirkungsprüfungen besteht das Problem, unter den Messbedingungen die optimalen Konzentrationen oder Aktivitäten der zu prüfenden Substanzen einzustellen. Unter realen Testbedingungen sind die sich daraus ergebenden Forderungen für die Probenvorbereitungen von z. T. unbekannten Substanzen nur mit hohem Aufwand einzuhalten.

Wirkungstests, die auf einem mehrstufigen Prozess beruhen (z. B. auf Basis ß-Galactosidase-Expression mit nachgeschalteter Farbreaktion), erfordern nach dem Stand der Technik eine mehrschrittige Arbeitsweise mit dem daraus folgenden erhöhten Aufwand für die Durchführung des Tests.

Geeignete Proben für Wirkungsprüfungen nach bekannten Verfahren sind Reinsubstanzen. In der Praxis liegen jedoch meistens Substanzgemische vor.

Hierzu wird in EP 588 139 beschrieben, wie die biologische Wirkung von Substanzen durch eine Kombination aus chromatographischer Auftrennung der zu testenden Substanzen in chromatographische Zonen mit einem anschließenden Test der biologischen Wirkung (der Toxizität) der einzelnen aufgetrennten Fraktionen, überprüft wird. Dazu werden die einzelnen Fraktionen in Kontakt mit Leuchtmikroorganismen gebracht, die durch eine lokale Änderung ihrer Biolumineszenz an den einzelnen Fraktionen die biologische Wirkung dieser Fraktion anzeigen.

Die Trennung der Substanzen in die Fraktionen erfolgt z.B. mittels Dünnschichtchromatographie (DC) oder Säulenchromatographie (HPLC). Im Falle der Dünnschichtchromatographie wird die DC-Platte mit einer Leuchtmikroorganismen-Suspension benetzt und die den einzelnen Fraktionen zugeordnete, lokale Biolumineszenz untersucht. Im Falle der Trennung mit Hilfe der Säulenchromatographie wird dem Eluat aus der chromatographischen Säule die Leuchtmikroorganismen-Suspension kontinuierlich zugemischt und die Biolumineszenz der Mischung gemessen.

In P. D. Shaw et al., Proc. Natl. Acad. Sci. USA, Vol. 94, 1997, 6036-6041 ist eine dünnschichtchromatographische Methode beschrieben, bei der die Detektion von Homoserinlacton über ein Reportergensystem in einer 3 mm starken Agarschicht als Nährmedium erfolgt. Dieses Verfahren wird ausschließlich für den analytischen Nachweis des Homoserinlactons eingesetzt. Das für den Nachweis von Homoserinlacton beschriebenen Detektionssystem beruht auf einem modifizierten *Agrobacterium tumefaciens* und Farbstoffbildung. Mit der hier beschriebenen hohen Schichtdicke können nur eine relativ geringe Abbildungsleistung und hohe Erfassungsgrenzen erreicht werden.

In WO 97/16569 ist die Wirkungsdetektion von Substanzen, die ausschließlich durch Festphasensynthese, z.B. an Polymerbeads erhalten werden, beschrieben. Zur Wirkungsdetektion werden enzymatische Assays und Bindungsassays aufgeführt. Eine allgemeine Anwendung auf Prüfsubstanzen beliebiger Herkunft wird nicht angesprochen. Auch die für die praktische Anwendung wichtigen Möglichkeiten zur direkten Kopplung mit chromatographischen Trenntechniken oder mit spektroskopischen Strukturaufklärungsmethoden sind mit dem in WO 97/16569 beschriebenen Verfahren nicht realisierbar.

WO 94/02515 offenbart ebenfalls die Freisetzung von Substanzen aus Polymerbeads sowie eine daran anschließende Wirkungsdetektion (Zell-basierte Assays). Auch hier werden keine allgemeineren Anwendungen angesprochen.

In EP 063 810 A1 wird eine spezielle Aüsfühningsform von Immunoassays für diagnostische Anwendungen beschrieben. Die Anmeldung zielt auf die Herstellung und Anwendung von Teststreifen für medizinisch relevante Tests.

In US 5,766,963 wird ein enzymatischer Assay mit einem Enzym als Sensor offenbart, bei dem Prüf-Substanzen durch Festphasensynthese auf feste Träger z.B. Beads aufgezoge und in Kontakt mit einer kolloidalen Matrix, z.B. Agarose gebracht werden. Unter Lichteinstrahlung diffundieren die Substanzen von den festen Trägern in die Matrix und bilden Zonen hoher Substanzkonzentration in der Nähe der Träger. In der Matrix kommen die Substanzen in Kontakt mit dem Enzym. Die Wechselwirkung von Substanz und Enzym erzeugt einen meßbaren photometrischen Effekt z.B. Fluoreszenzemission. Diese Fluoreszenzemission kann über eine große Fläche mit einem Fluorimeter oder einer CCD-Kamera detektiert werden.

Die Aufgabe der Erfindung ist es, eine im Vergleich zum Stand der Technik einfachere, empfindlichere, schnellere, breiter einsetzbare und mit geringerem Artefaktrisiko behaftete Möglichkeit zur Erfassung der biologischen Wirkung von Substanzen zu finden

Die Lösung der erfindungsgemäßen Aufgabe ist eine Sensorschicht zur Erfassung der biologischen Wirkung von Substanzen, die in Kontakt mit der Probe gebracht wird, bestehend aus einer diffüsionskontrollierenden Matrix und darin suspendierten Sensoren, wobei in 50 ml Sensorschichtmasse 2 bis 8 ml, bevorzugt 3 bis ml, Reportergenzellsuspension enthalten sind und die Reportergenzellen biolumineszent sind.

Die Matrix kann dabei ein Nebenvalenzgel, z. B. Agarose, ein Polymergel, z.B. Acrylat oder eine viskose Lösung, z.B. Polyethylenglykol in Wasser sein. Agar ist wegen des relativ hohen Gelierpunktes insbesondere bei temperaturlabilen Sensoren wenig geeignet.

Die Sensorschicht bildet eine Diffusionsbarriere für die mit ihr in Kontakt befindlichen Substanzen. Verschiedene Stoffe diffundieren unterschiedlich schnell und unterschiedlich weit in die Sensorschicht hinein, da der Massentransport in der Sensormatrix substanzspezifisch unter anderem abhängig von Polarität und Molekülgröße verläuft. Dadurch bildet sich innerhalb der Sensorschicht ein Konzentrationsgradient für die einzelnen zu prüfenden Fraktionen oder Substanzen aus. Für jedes Detektionsprinzip mit dem zugehörigen Sensor ist in einem bestimmten Abstand vom Träger eine optimale Konzentration der zu prüfenden Substanz oder Fraktion vorhanden. Innerhalb des Profils kann auch die konzentrationsabhängige Wirkung beobachtet werden. Außerdem werden störende Substanzen wie Verunreinigungen durch den Diffusionsprozess räumlich innerhalb der Sensorschicht von wirksamen Substanzen abgetrennt.

Darüber hinaus können in die Sensorschicht Zusätze eingebracht werden, die den Detektionsprozess direkt kontrollieren, indem sie die Nachweisempfindlichkeit, die Selektivität und die Kinetik der Sensorschicht beeinflussen. Ein solcher Zusatz ist z. B. ein Puffer zur Regulierung des Vitalitätsstatus von Sensorzellen.

Die diffusionskontrollierende Sensorschicht kann auch Indikatorsubstanzen (z. B. pH-Indikatoren, Redoxfarbstoffe) aufnehmen, die ortsauflösende Aussagen zu pH-Werten oder Redoxeigenschaften auf Oberflächen von Untersuchungsobjekten ermöglichen.

Der Zusatz von Biolumineszenzsubstraten, die in bestimmten Prüfverfahren eine Rolle spielen, in die Sensorschicht erlaubt es, mehrstufige Detektionsprozesse in der Sensorschicht in einem Arbeitsschritt auszuführen.

Die Schichtdicke der Sensorschicht beträgt bevorzugt 0,1 bis 10 mm, besonders bevorzugt 0,5 bis 3 mm, ganz besonders bevorzugt 0,5 bis 0,8 mm.

Die eingesetzte Reportergenzellsuspension hat bevorzugt eine optische Dichte von 0,6 bis 1,4 bei 660 nm Wellenlänge.

Die Sensorschicht selbst kann aus mehreren Schichten aufgebaut sein. Dabei dienen die einzelnen Schichten verschiedenen Zwecken und können entsprechend ihrem Zweck unterschiedlich dick sein. Die einzelnen Schichten können folgende Funktion haben:
- Aufnahme unterschiedlicher Reportergenzelllinien für multiple Detektion
- Aufnahme von Zusätzen zur Steuerung und Unterstützung des Detektionsprozesses
- Wirkung als Diffusionsbarriere oder Substanz-selektive Filterschicht.

Es können auch mehrere, verschiedene Reportergenzelllinien mit unterschiedlicher Wirkungsspezifität in die Matrix suspendiert werden, um so verschiedene biologische Wirkungen simultan zu erfassen. Es ergibt sich so eine Multisensorschicht. Um zu unterscheiden welche Reportergenzelllinien in einer solchen Multisensorschicht eine biologische Wirkung zeigt, müssen die von den einzelnen Reportergenzelllinien ausgesandten Signale unterschiedlich sein. Beispiele hierfür sind eine Multisensorschicht mit mehreren Reportergenzellinien, die unterschiedliche biologische Aktivitäten mit unterschiedlichen Signalen anzeigen. Statt mehrerer verschiedener Reportergenzelllinien können auch Reportergenzellinien, die mehrere unterschiedliche Wirkungen simultan anzeigen, verwendet werden. Die Signale der Multisensorschicht lassen sich parallel auswerten.

Die Lösung der erfindungsgemäßen Aufgabe ist außerdem ein Verfahren zur Prüfung der biologischen Wirksamkeit von Substanzen, mit den Merkmalen des Anspruchs 10. Die Probe wird zunächst auf oder in die Oberfläche eines Trägers gebracht, sofern sie nicht bereits Bestandteil eines Trägers ist. Als Träger kann entweder die Sensorschicht selber dienen oder ein zusätzlicher Träger. Sofern die Sensorschicht nicht selbst als Träger dient, wird der Träger dann mit der erfindungsgemäßen Sensorschicht belegt. Anschließend wird die Wirkung der Substanz auf die Reportergenzelllinien in der Sensorschicht bestimmt.

Als Träger können außer der Sensorschicht selber glatte, strukturierte oder poröse Objekte aus Glas, Kunststoffen, Metall oder aus anderen organischen oder anorganischen Materialien und Werkstoffen dienen. Insbesondere sind Papier, Membranen, Filme, Folien oder Polymerperlen geeignet. Es kann auch direkt biologisches Material wie Gewebeschnitte oder Pflanzenblätter als Probe dienen, so dass hier die Probe gleich Bestandteil des Trägers ist.

Die Sensorschicht kann auf den Träger aufgetragen werden durch Gießen, Tauchen, Walzen, Sprühen oder als Film.

Wenn die Sensorschicht selbst als Träger dient, wird die zu testende Substanz direkt auf die Sensorschicht aufgebracht z.B. durch Mikrodosiersysteme oder drucktechnische Verfahren, bei denen Substanzen auf die Schicht übertragen werden. Ein drucktechnisches Verfahren besteht beispielsweise darin die Nadeln eines Stempels in die zu testenden Substanzen zu tauchen, die sich beispielsweise in den Wells einer Mikrotiterplatte befinden. Dabei benetzt jede Einzelsubstanz eine Nadelspitze. Der Stempel mit den Nadeln wird dann in die Sensorschicht gepresst. Alternativ können auch die Probensubstanzen auf Papier aufgetragen werden und dieses Papier mit der durch die Probensubstanzen beschichteten Seite auf die Sensorschicht gedrückt werden.

Es kann vor der Bestimmung der Wirkung der in der Probe enthaltenen Substanzen auf die Reportergenzellen ein Inkubationsschritt erfolgen. Hierzu wird die Sensorschicht oder der mit der Sensorschicht belegte Träger entsprechend den Anforderungen der eingesetzten Zellen unter definierten Bedingungen bezüglich Temperatur, Feuchtigkeit und Begasung eine vorgegebene Zeit gelagert. Danach wird erst die Wirkung der zu testenden Substanz auf die Reportergenzellen bestimmt.

Das erfindungsgemäße Verfahren gestattet es, Anreicherungsverfahren direkt mit dem Wirkungstest zu verbinden. Anreicherungen sind z. B. nötig, wenn es sich um schwach wirksame Substanzen handelt. Die Inhaltsstoffe einer Probenlösung können durch unspezifische oder durch spezifische Adsorption an geeigneten Trägern wie Membranen, Ionenaustauschmatrices, Affinitätsmatrices, Dünnschichtchromatographieplatten oder Papier angereichert werden. Das Anreichern kann durch direkten Kontakt des Trägers mit einem hinreichend großen Volumen der Probelösung erreicht werden. Hierzu können auch spezielle Probenaufgabetechniken der Chromatographie mit anreichernder Wirkung, z. B. Konzentrierungsschichten aus der Dünnschichtchromatographie oder steile Lösemittelgradienten eingesetzt werden. Die auf der Trägermatrix angereicherten und immobilisierten Substanzen können nach Belegen mit der aktiven Sensorschicht direkt auf ihre biologische Wirkung geprüft werden.

Die Wirkung der Substanzen der Probe auf die Reportergenzellen in der Sensorschicht wird bevorzugt mit Hilfe bildgebender Verfahren wie photographische Verfahren, Video Imaging oder auch als Handzeichnung festgehalten. Typische Reaktionen von Reportergenzellen auf eine Substanz sind z. B. die Induktion oder die Löschung der Lichtemission aus Biolumineszenzprozessen. Die biologische Aktivität wird für die Positionen auf dem Träger, an denen sich eine bestimmte Substanz befindet, aufgrund des jeweiligen Sensormechanismus angezeigt.

Biolumineszenz gestattet die mehrfache Beobachtung der detektierten Wirkung zu verschiedenen Zeitpunkten, so dass auch detaillierte kinetische Resultate zum Verlauf der Wirkung erhalten werden. Bevorzugt werden Nachweisverfahren über die Messung von Lichtemission gegenüber Messungen der spektralen Änderung der Lichtabsorption, da hier deutlich höhere Empfindlichkeiten zu erreichen sind und bei der Messung von Lichtemission nach wesentlich kürzerer Zeit Signale nachweisbar sind.

Die Auswertung der Bilddaten kann qualitativ im Sinne einer Ja/Nein-Aussage zur biologischen Aktivität und quantitativ zur Bewertung von Wirkungshöhen und der räumlichen Verteilung der Aktivität erfolgen. Hierzu können Bildverarbeitungsprogramme oder auch visuell vergleichende Verfahren eingesetzt werden, die jeweils mit bekannten Referenzwirkungen kalibriert werden.

Bei der chromatographischen Trennung der Fraktionen mittels einer Chromatographiesäule wird das Eluat aus der Chromatographiesäule fortlaufend entweder kontinuierlich oder in Intervallen auf verschiedene Stellen des Trägers aufgebracht, z. B. in Form einer Serie von Spots oder durch Sprühen.

Die Auftrennung in die Fraktionen kann mit einer Strukturuntersuchung der in den Fraktionen vorliegenden Einzelsubstanzen gekoppelt werden, so dass eine durch die Sensorschicht nachgewiesene biologische Wirkung einer Fraktion mit der Strukturenformation über die Einzelsubstanz verknüpft werden kann. Die Strukturinformation wird aus einer spektroskopischen Untersuchung erhalten. Vorzugsweise werden die Ergebnisse der spektroskopischen Untersuchung erst ausgewertet, nachdem mit der Sensorschicht die biologische Wirkung der Einzelsubstanzen bestimmt worden ist. Die Auswertung der Ergebnisse der spektroskopischen Untersuchung erfolgt bevorzugt nur für die biologisch aktiven Einzelsubstanzen.

Erfolgt die chromatographische Trennung der Fraktionen über Säulenchromatographie, so wird ein Teil des Eluats fortlaufend entweder kontinuierlich oder in Intervallen auf verschiedene Stellen des Trägers aufgebracht, während gleichzeitig ein anderer Teil des Eluats abgezweigt und durch ein Massenspektrometer oder ein NMR-Spektrometer oder ein IR-Spektrometer spektroskopiert wird (z. B. mittels der chromatographischen Kopplungstechniken HPLC/MS, HPLC/NMR, HPLC/IR) Bevorzugt wird das Eluat aus der Chromatographiesäule vor Abzweigung des zu spektroskopierenden Anteils durch einen UV-Detektor detektiert.

Erfolgt die chromatographische Trennung des Substanzgemisches in einzelne Substanzzonen mittels Dünnschichtchromatographie oder Elektrophorese auf dem Träger, so können z. B. von den einzelnen Substanzzonen, durch Matrix Assisted Laser Desorption Ionization (MALDI)-Massenspektroskopie oder Raman-Spektroskopie oder andere spektroskopische Verfahren (UV-VIS, IR) Spektren aufgenommen werden, bevor der Träger mit der Sensorschicht belegt wird. Die Lösung der erfindungsgemäßen Aufgabe besteht weiterhin in einer Vorrichtung bestehend aus einer erfindungsgemäßen Sensorschicht, die in Kontakt mit der zu untersuchenden Substanz steht und einem bildgebenden System in dessen Detektionsbereich sich ein Teil oder die gesamte Sensorschicht befindet.

Die Reportergenzellen der Sensorschicht zeigen ihre Aktivität durch Emission oder Löschung von Lichtemission an, die dann durch ein entsprechendes bildgebendes System detektiert wird.

Die erfindungsgemäße Sensorschicht, das erfindungsgemäße Verfahren und die erfindungsgemäße Vorrichtung bieten gegenüber dem Stand der Technik eine Reihe von Vorteilen, insbesondere für Zell-basierte Wirkungsprüfungen:
1. Die Sensorschicht steuert sowohl durch Diffusionskontrolle wie auch über regulierende und unterstützende Zusätze den Detektionsprozess. Darüber hinaus erlaubt der Aufbau der Sensorschicht die gleichmäßige Beschichtung unterschiedlicher Materialien (Glas, Kunststoff, Metall) und Oberflächen (glatt, rauh, porös). Weiterhin wird die Sedimentation der Sensororganismen während des Detektionsprozesses vermieden.
2. Bei Untersuchungen bioaktiver Substanzen besteht häufig die Gefahr, dass der Test gestört wird, wenn die Konzentration der zu testenden Substanz zu hoch ist. Dieses Artefaktrisiko ist durch den großen erfassten Konzentrationsbereich in der diffusionskontrollierenden Sensorschicht deutlich verringert. Der Konzentrationsgradient einer Substanz in der Sensorschicht erlaubt Zonen biologischer Aktivität unterhalb cytotoxischer Konzentrationen von Substanzen zu erfassen. Dies ist vorteilhaft für Wirkungstests von Substanzen mit unbekannten Prüfkonzentrationen. Der Aufwand für die Bestimmung und Standardisierung der Substanzkonzentrationen kann reduziert werden oder entfallen.
3. Substanzgemische, die cytotoxische oder andere, mit Wirkungstests interferierende Komponenten enthalten, können im Vergleich zu konventionellen Techniken mit geringerem Risiko falsch negativer Ergebnisse auf biologische Aktivität geprüft werden. Die diffusionskontrollierende Sensorschicht erzeugt für unterschiedliche Substanzen unterschiedliche Konzentrationsprofile und trennt so die störenden von den zu testenden Bestandteilen des Substanzgemischs.
4. Da sich auf einem Träger verschiedene Substanzen räumlich getrennt befinden, z. B. in chromatographischen Zonen, besteht in der konventionellen Technik die Gefahr, dass sich, insbesondere bei langen Inkubationszeiten die Substanzen auf verschiedenen Positionen des Trägers vermischen. Die diffusionskontrollierende Sensorschicht verhindert eine zu starke Diffusion selbst bei langen Inkubationszeiten, so dass die Zonen wirksamer Substanzen auf Trägern auch nach langen Inkubationszeiten über ihre Wirkung erkannt werden können.
5. Das erfindungsgemäße Verfahren erlaubt eine hohe räumliche Auflösung.
6. Beim Einsatz einer Multisensorschicht lassen sich in kurzer Zeit Wirkungen von Substanzen auf eine Vielzahl von Sensoren untersuchen. Es können leicht Wirkungsprofile von Wirkstoffen aufgestellt werden.
7. Die direkte Kombination der Technik die Substanzgemische auf dem Träger in Fraktionen zu trennen und die Fraktionen spektroskopisch auf die Struktur der Einzelsubstanzen zu untersuchen mit der Detektion der biologischen Wirksamkeit der Einzelsubstanzen durch die Sensorschicht erlaubt es, Aussagen zur chemischen Struktur unbekannter, biologisch aktiver Substanzen unmittelbar aus Gemischen zu erhalten.

Das erfindungsgemäße Verfahren kann zur Untersuchung von biologischen Wirkungen und der Aufstellung von Bioaktivitätsprofilen in "Drug-Discovery-Progmmmen" verwendet werden.

### Figuren und Beispiele

Die Figuren zeigen:
- Fig. 1: Schematischer Aufbau einer erfindungsgemäßen Vorrichtung.
- Fig. 2: Ergebnis einer Messung der örtlichen Verteilung von biologischer Aktivität auf einer Oberfläche mit der erfindungsgemäßen Sensorschicht.
- Fig. 3: Vergleich zwischen konventionellem und erfindungsgemäßem Verfahren bei der Messung an einem Substanzgemisch mit toxischen Bestandteilen.
- Fig. 4: Versuchsaufbau zur Kopplung von chromatographischer Trennung und Spektroskopie

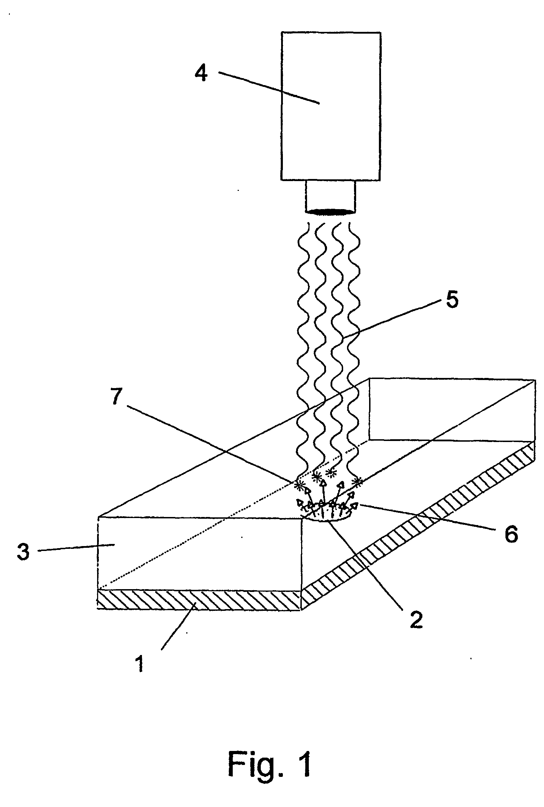

Fig. 1 zeigt schematisch eine erfindungsgemäße Vorrichtung. Auf dem Träger 1 befindet sich als Probe eine adsorbierte Substanz 2. Auf dem Träger 1 mit der Substanz 2 liegt die diffusionskontrollierende Sensorschicht 3. Oberhalb der Sensorschicht 3 befindet sich ein bildgebendes System 4, das das optische Signal 5 detektiert. Die auf dem Träger 1 befindliche Substanz 2 diffundiert 6 in die Sensorschicht 3 und löst an den Sensoren 7 das optische Signal 5 aus.

### Beispiel 1

Beispiel 1 zeigt eine Abbildung biologisch aktiver Strukturen auf einem festen Träger mit der erfindungsgemäßen Sensorschicht.

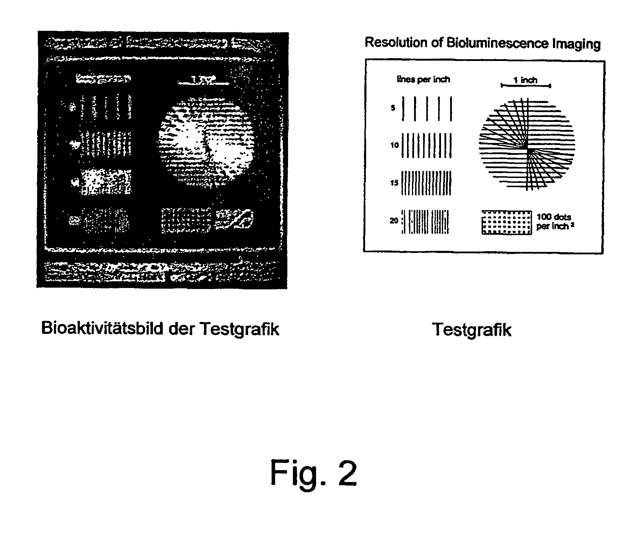

Um die Abbildungsleistung einer Sensorschicht zu untersuchen, wurde die wirkungsaktive Substanz Ciprofloxacin mittels eines Inkjet-Printers (HP DeskJet 870 Cxi) in Form einer Testgrafik auf einen Träger aufgebracht (Fig. 2, rechte Seite). Als Träger diente eine mit Kieselgel 60 beschichtete Aluminiumfolie von Merck (Art. Nr. 1.05553). Zur Wirkungsdetektion enthielt die Sensorschicht gentechnisch hergestellte Reportergenzellen, die die biologische Aktivität des Ciprofloxacins durch Biolumineszenz wirkungsspezifisch anzeigen. Die durch den Ciprofloxacin-Auftrag festgelegte örtliche Wirkungsverteilung auf dem Papierträger wurde über die in der Sensorschicht induzierte Biolumineszenz mit einem Video-Imaging-System registriert.

Das Ergebnis ist in Fig. 2, linke Seite dargestellt und zeigt für den gesamten Biolumineszenz-Imagingprozess eine grafische Auflösung von ca. 20 lpi (lines per inch). Dies reicht aus, um z. B. Wirkungsprüfungen als Tüpfeltests mit einer Spotdichte von mehr als 25 Spots/cm² auszuführen.

Detailangaben und die experimentellen Bedingungen zu Beispiel 1 sind nachfolgend angegeben:

### 1. Reportergenzellen

Die Reportergenzelle besteht aus dem *Escherichia coli* Stamm SM101, der das rekombinante Plasmid pEBZ181 trägt. Dieses Plasmid kodiert eine Fusion zwischen dem *recA*-Promotor aus *E. coli* und den Strukturgenen des bakteriellen Leuchtens aus *Vibrio fischeri* (*lux*-Gene *C, D, A, B, E* und *G*). Zur Konstruktion des Plasmides wurde das *recA*-Promotor-tragende *Bam* H I-Fragment aus dem Plasmid pUA80 [Barbe J, Fernandez de Henestrosa AR, Calero S, and Gibert I (1991) Chromogenic Method for rapid isolation of *recA*-like mutants of Gram-negative bacteria. J. Bacteriol. 173: 404-406] in ein Derivat des Vektors pEBZ112 [Peitzsch N, Eberz G, and Nies D (1998) *Alcaligenes eutrophus* as a bacterial chromate sensor. Appl. Environm. Microbiol. 64: 453-458], das inseriert in der *Nco* I Stelle des *luxG*-Gens eine *lacZ*-Kassette [Becker A (1993) Analyse der Succinoglycan-Biosyntheseregion von *Rhizobium meliloti* 2011: Untersuchungen zur Identifizierung des bakteriellen Infektionssignals in der Symbiose mit Luzerne. Doktorarbeit, Universität Bielefeld, Deutschland] trägt, kloniert. Plasmid pEBZ181 wurde über Standardmethoden [Sambrook J, Fritsch EF, and Maniatis T (1989) Molecular cloning: A laboratory manual (2nd edn), Cold Spring Harbor Laboratory Press] in den *Escherichia coli* Stamm SM101 (erhalten vom E. coli Genetic Stock Center, Yale University, New Haven, USA) transformiert und für Bioimaging-Versuche eingesetzt.

Die Behandlung von Bakterien mit antibakteriellen Agenzien wie 4-Chinoloncarbonsäuren führt zur Induktion des sogenannten SOS-Reparaturmechanismus [Walker CG (1984) Mutagenesis and inducible responses to deoxyribonucleic acid damage in *Escherichia coli*. Microbiol. Rev. 48: 60-93; Phillips I, Culabras E, Moreno F, and Baquero F (1987) Induction of the SOS response by new 4-quinolones. J. Antimicrob. Chemother. 20: 631-638; Piddock LJV, and Wise R (1987) Induction of the SOS response in *Escherichia coli* by 4-quinone antimicrobial agents. FEMS Microbiol. Lett. 41: 289-294]. Das RecA-Protein ist ein Hauptregulator dieses Reparaturmechanismus, wobei die SOS-Induktion zu einer verstärkten Expression des *recA*-Gens führt. Die Messung der Synthese des RecA-Proteins [Little JW and Mount DW (1982) The SOS regulatory system of *Escherichia coli.* Cell 29: 11-22; Witkin EM (1976) Ultraviolet mutagenesis and inducible DNA repair in *Escherichia coli*. Bacteriol. Rev. 40: 864-907] oder von *recA*-Reportergenfusionen [Nunoshiba T and Nishioka H (1991) *Rec-lac* test for detecting SOS-inducing activity of environmental genotoxic substances. Mutation Res. 254:71-77] ist eine Möglichkeit die SOS-Induktion und folglich die Wirkung von 4-Chinoloncarbonsäuren zu messen. Da der Stamm *E. coli* SM101 (pEBZ181) ein Plasmid mit einer *recA-lux* Reportergenfusion trägt, eignet er sich zum Nachweis von 4-Chinolon-carbonsäuren oder anderen SOSinduzierenden Verbindungen. Die Gegenwart solcher Verbindungen führt zu einer verstärkten Expression der Luciferasegene und damit zur einer Stimulierung der Biolumineszenz.

### 2. Sensorschichtmasse

Die Beschichtungsmasse setzte sich folgendermaßen zusammen:
- 32 ml 1 % Agarose (Agarose MP Bochringer Mannheim GmbH Art. Nr. 1388983); diese niedrig schmelzende Agarose erlaubt es, temperaturempfindliche Reportergenzellen bei Temperaturen unter 40 °C ohne Schädigung zu suspendieren.
- 4 ml LB Medium 200 g/l (GIBCO BRL Art. Nr. 12780-052); der Zusatz von LB-Medium dient zur Aufrechterhaltung der vitalen Funktionen der Reportergenzellen während der Inkubationszeit.
- 4 ml Bakteriensuspension in LB-Medium 20 g/l; damit ergab sich eine optische Dichte von 1,2 bei 660 nm Wellenlänge; über das Volumen der zugesetzten Bakteriensuspension wird die Zelldichte, d. h. die Dichte der Sensoren in der Sensorschicht gesteuert, um das Signal-Rausch-Verhältnis und die Ortsauflösung zu optimieren.
- 10 ml Wasser; dieser Zusatz regelt die Viskosität der Gießmasse und die mechanische Stabilität der Sensorschicht nach dem Aushärten.

### 3. Testgrafik

Die Testgrafik wurde mit Corel Draw (Version 8) erstellt. Die Linienstärke beträgt für die Linienraster 0,1 mm. Die Dot-Matrix enthält in 1/2 Inch² 50 quadratische Dots (Kantenlänge 1 mm). Die Dots wurden mit einer Farbsättigung von 50 % gedruckt, alle übrigen Elemente der Testgrafik wurden mit 100 % Farbsättigung gedruckt.

### 4. Ciprofloxacin-Auftrag auf Kieselgel-beschichtete Aluminiumfolie

42 ml einer Lösung von 120 mg Ciprofloxacinhydrochlorid-Monohydrat in Wasser wurden in eine vorher entleerte Tintenpatrone des Inkjet-Printers HP DeskJet 870 Cxi gegeben. Die unter 3. beschriebene Testgrafik (Fig. 2, rechte Seite) wurde mit dem Inkjet Printer auf die Kieselgel-Schicht der Aluminiumfolie (Merck Art. Nr. 1.05553) gedruckt.

### 5. Auftrag der Sensorschicht und Inkubation

Die Aluminiumfolie wurde unter einem Stickstoffstrom getrocknet, in einen passenden Edelstahlrahmen mit erhöhtem Rand eingesetzt, auf einem Nivelliertisch waagerecht positioniert und mit der unter 2. erhaltenen Sensorschichtmasse gleichmäßig begossen, so dass eine Schichtdicke von 2 mm erreicht wurde. Nach Aushärten der Sensorschicht bei Raumtemperatur wurde der beschichtete Träger bei 28 °C für 60 Minuten inkubiert.

### 6. Videoimaging

Der nach 5. inkubierte Träger wurde mit einem Videoimaging-System (Molecular Light Imager NightOWL" von EG&G Berthold) gemäß Bedienungsanleitung vermessen. Die Aufnahmezeit betrug 60 s, die Kameraposition wurde auf ein Bildformat von 10 x 20 cm optimiert. Zur Darstellung der Ergebnisse wurden die erhaltenen Image-Daten in TIFF-Files konvertiert und anschließend mit geeigneten Grafikprogrammen (Corel Draw Version 8, Corel Photo Paint Version 8 oder Photoshop Version 4.0) formatiert, beschriftet und über einen Laserprinter (HP LaserJet 5) ausgegeben. In Fig. 2 wird das Biolumineszenz-Image, das die Wirkung des auf die Kieselgel/Aluminiumfolie gedruckten Ciprofloxacins abbildet, zusammen mit der Original-Testgrafik gezeigt.

### Beispiel 2

Beispiel 2 zeigt eine Anwendung der Sensorschicht für Aktivitätstests in Gegenwart cytotoxischer Substanzen und einen Vergleich mit einem analogen Mikrotiterplattentest.

Im Gegensatz zu üblichen Mikrotiterplatten-Formaten kann die diffusionskontrollierende Sensorschicht für Tests von Substanzgemischen, die störende Komponenten enthalten, eingesetzt werden, denn die Störkomponenten werden durch Diffusionsund Adsorptionsprozesse in situ abgetrennt, so dass das Risiko für falsch negative Resultate herabgesetzt wird. Am Beispiel von Bioaktivitätstests mit dem SOS-Reportergensystem (siehe Beispiel 1) kann die Überlegenheit der Sensorschicht im Vergleich zu Mikrotiterplattenformaten belegt werden.

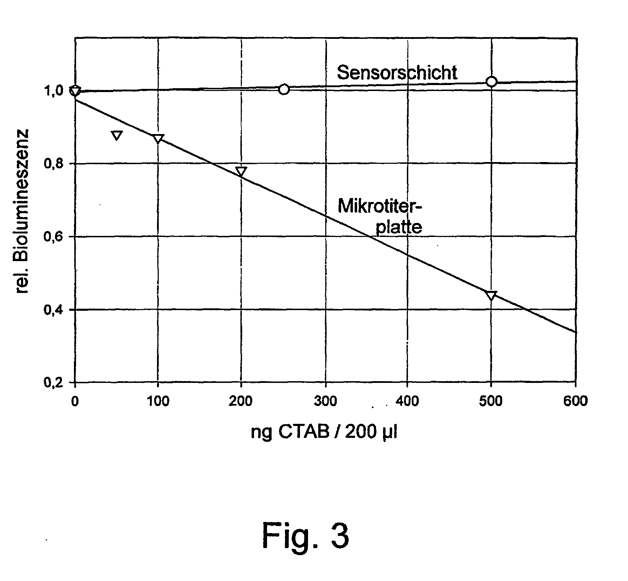

In beiden Testformaten diente Ciprofloxacin zur wirkungsspezifischen Induktion der SOS-Antwort. Die in dem hier verwendeten Reportergensystem stimulierte Biolumineszenz lieferte das Read-Out-Signal, das durch Videoimaging erfasst wurde. Um die Toleranz der beiden Bioassayformate gegenüber störenden Substanzen zu prüfen, wurden Tests in Gegenwart von cytotoxischem Cetyltrimethylammoniumbromid (CTAB) ausgeführt. Dabei zeigte sich, dass für das Sensorschicht-Format die relative Biolumineszenz als Maß der selektiven Stimulation durch Ciprofloxacin bis zu CTAB-Konzentrationen von 500 ng/200 µl konstant blieb, während das Mikrotiterplattenformat in diesem Bereich bereits eine Biolumineszenzminderung von über 50 % aufwies (Fig. 3).

Detailangaben und die experimentellen Bedingungen zu Beispiel 2 sind nachfolgend aufgeführt:

### 1. SOS-Reportergensystem

Es wurde das gleiche SOS-Reportergensystem verwendet wie in Beispiel 1.

### 2. Mikrotiterplatten-Tests

Fünf Testlösungen wurden in einer 96-Well-Mikrotiterplatte (Dynatech Microlite) angesetzt. In jeweils 200 µl Volumen waren enthalten:
94 µl 2 % LB Medium (GIBCO BRL Art. Nr. 12780-052)
16 µl Reportergenzellsuspension (OD: 1,2 bei 660 nm) in 2 % LB Medium
90 µl Lösung der Substanzen 1. - 5. in Wasser
   1. 25 ng Ciprofloxacinhydrochlorid-Monohydrat
   2. 25 ng Ciprofloxacinhydrochlorid-Monohydrat + 50 ng CTAB
   3. 25 ng Ciprofloxacinhydrochlorid-Monohydrat + 100 ng CTAB
   4. 25 ng Ciprofloxacinhydrochlorid-Monohydrat + 200 ng CTAB
   5. 25 ng Ciprofloxacinhydrochlorid-Monohydrat + 500 ng CTAB

Die Mikrotiterplatte wurde 60 min bei 28°C inkubiert und darauf mit dem Videoimagingsystem Molecular Light Imager NightOWL" von EG&G Berthold vermessen. Für die einzelnen Wells wurden jeweils die mittleren Grauwerte bestimmt. Die relative Biolumineszenz wurde durch Division dieser Grauwerte durch den Wert für die CTAB-freien Referenzlösung ermittelt. Für zwei Messreihen wurden die Mittelwerte der relativen Biolumineszenzintensität in Abhängigkeit von der CTAB-Konzentration bestimmt:

| CTAB [ng/200µl] | rel. Biolumineszenz |
|---|---|
| 0 | 1,00 |
| 50 | 0,88 |
| 100 | 0,87 |
| 200 | 0,78 |
| 500 | 0,44 |

Zum Vergleich sind die Ergebnisse in der untenstehenden Abbildung den Resultaten für das Sensorschichtformat gegenübergestellt.

### 3. Aktivitätsmessungen mit der Sensorschicht

Zur Erfassung der Ciprofloxacinwirkung mit der Sensorschicht wurden auf eine Dünnschichtchromatographieplatte (Merck Art. Nr. 1.15445 Si 60 F₂₅₄ₛ) mittels Einmalmikropipetten die folgenden Testlösungen aufgegeben:
1. 1 µl wässrige Lösung von 10 ng Ciprofloxacinhydrochlorid-Monohydrat
2. 1 µl wässrige Lösung von 10 ng Ciprofloxacinhydrochlorid-Monohydrat + 50 ng CTAB
3. 1 µl wässrige Lösung von 10 ng Ciprofloxacinhydrochlorid-Monohydrat + 100 ng CTAB

Die unter einem Stickstoffstrom getrockneten DC-Platten wurden mit einer Suspension der lumineszenten Sensorbakterien (siehe Beispiel 1) in 0,6 % Agarose mit einer Schichtdicke von ca. 2 mm auf einem Nivelliertisch planar beschichtet. Danach wurden die DC-Platten für 60 min bei 28°C inkubiert. Anschließend erfolgte die Detektion der induzierten Biolumineszenz durch Imaging mit dem hochauflösenden CCD Low Light Imaging System "Molecular Light Imager NightOWL" von EG&G Berthold. Imaging-Bedingungen: Aufnahmezeit 60 s; Kameraposition auf DC-Plattenformat von 10 x 20 cm optimiert. Für die lumineszierenden Spots wurden die mittleren Grauwerte bestimmt. Um den Vergleich mit den oben beschriebenen Resultaten für das Mikrotiterplattenformat zu führen, wurden aus den auf die Dünnschichtplatte aufgegebenen Substanzmengen die analogen Konzentrationen ermittelt. Für diese Abschätzung wurde eine Diffusion der Substanzen im Sensorschichtvolumen oberhalb der Substanzspots (Spotdurchmesser 5 mm, Schichtdicke 2 mm) angenommen. Zwei Meßreihen lieferten die folgenden relativen Biolumineszenzintensitäten (Mittelwerte) in Abhängigkeit von der CTAB-Konzentration:

| CTAB [ng/200µl] | rel. Biolumineszenz |
|---|---|
| 0 | 1,000 |
| 250 | 1,002 |
| 500 | 1,023 |

### 4. Vergleich der Ergebnisse

Der Vergleich der Messungen mit beiden Formaten zeigt, dass im Fall der Sensorschicht die Bioaktivitätsmessung durch den CTAB-Zusatz nicht beeinträchtigt wurde, während beim Mikrotiterplattenformat ein deutlicher Abfall (ca. 40 % des Ausgangswerts) der Biolumineszenz auftrat. Diese Resultate sind in Fig. 3 grafisch gegenübergestellt.

### Beispiel 3

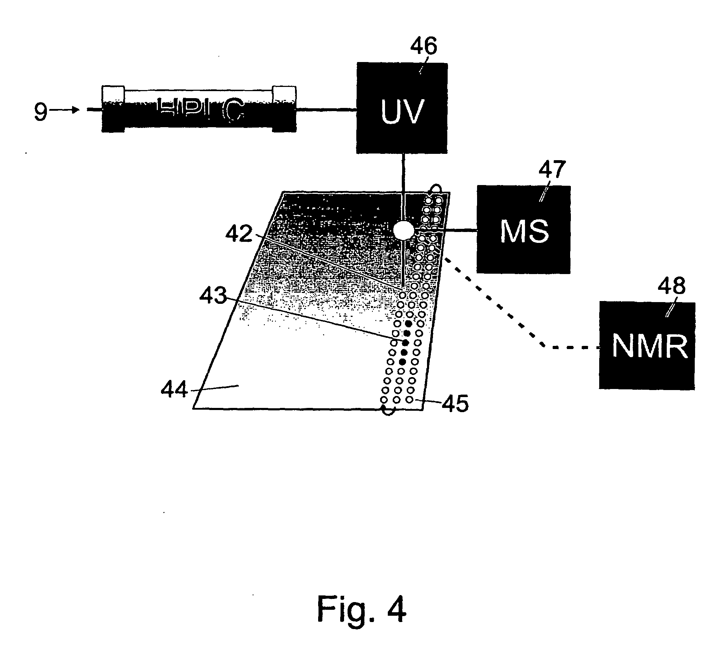

Fig. 4 stellt schematisch dar, wie durch Säulenchromatographie ein Substanzgemisch in Fraktionen aufgetrennt wird und gleichzeitig spektroskopische Daten zu den einzelnen Fraktionen gewonnen werden.

Die Probe 9 wird durch Säulenchromatographie (HPLC) getrennt. Das Eluat aus der Chromatographiesäule wird durch einen UV-Detektor 46 detektiert. Über ein Split-System wird ein Teil des Eluats auf einen Träger 44 durch Sprühen oder Spotten aufgebracht 42. Beginnend an einer Startposition 45 wird das Säulenchromatographie-Eluat rasterförmig auf den Träger 44 gegeben, so dass alle Substanzen aus dem Säulenchromatographie-Lauf dort räumlich getrennt deponiert werden. Ein anderer Teil des Eluats wird gleichzeitig in ein Massenspektrometer 47 oder ein NMR-Spektrometer 48 abgezweigt und dort spektroskopiert (HPLC/MS- bzw. HPLC/NMR-Kopplung). Nachdem alle Spots auf den Träger 44 aufgebracht sind, wird der Träger 44, gegebenenfalls nach einer Trocknung, mit der Sensorschicht belegt. Die Sensorschicht zeigt die Positionen der biologisch aktiven Fraktionen 43 an. Durch Korrelation mit den Säulenchromatographie-Elutionszeiten werden die Massenspektren oder NMR-Daten den einzelnen Spots eindeutig zugeordnet. Damit liegen detaillierte Strukturinformationen zu den wirksamen Substanzen nach Beendigung der biologischen Wirkungssprüfung sofort vor.

## Patentansprüche

1. Sensorschicht (3) zur Erfassung der biologischen Wirkung von Substanzen (2), wobei die Sensorschicht (3) aus einer diffusionskontrollierenden Matrix und darin suspendierten Sensoren (7) besteht, dadurch gekennzeichent, dass in 50 ml Sensorschichtmasse 2 bis 8 ml, bevorzugt 3 bis 5 ml, Reportergenzellsuspension enthalten sind und die Reportergenzellen biolumineszent sind.

2. Sensorschicht(3) nach Anspruch 1, **dadurch gekennzeichnet, dass** die Matrix ein Gel wie Agarose, Polyacrylate oder eine viskose Lösung ist.

3. Sensorschicht (3) nach Anspruch 1 oder 2, **dadurch gekennzeichnet, dass** mehrere verschiedene Arten von Reportergenzellen oder eine Art von Reportergenzellen, die verschiedene biologische Wirkungen anzeigen kann, in der Sensorschicht (3) suspendiert sind.

4. Sensorschicht (3) nach einem der Ansprüche 1 bis 3, **dadurch gekennzeichnet, dass** die Sensorschicht (3) Zusätze enthält, die den Detektionsprozess kontrollieren oder unterstützen

5. Sensorschicht (3) nach Anspruch 4, **dadurch gekennzeichnet, dass** die Zusätze Puffer für die Regulierung des Vitalitätsstatus von Reportergenzellen sind.

6. Sensorschicht (3) nach einem der Ansprüche 1 bis 5, **dadurch gekennzeichnet, dass** die Sensorschicht (3) Biolumineszenzsubstrate enthält.

7. Sensorschicht (3) nach einem der Ansprüche 1 bis 6, **dadurch gekennzeichnet, dass** die Sensorschicht (3) aus mehreren Teilschichten besteht, wobei die Teilschichten unterschiedlich dick sein können und sich durch Art und Menge von Reportergenzellen und/oder Zusätzen unterscheiden.

8. Sensorschicht (3) nach einem der Ansprüche 1 bis 7, **dadurch gekennzeichnet, dass** die Reportergenzellsuspension eine optische Dichte von 0,6 bis 1,4 bei 660 nm hat.

9. Sensorschicht (3) nach einem der Ansprüche 1 bis 8 **dadurch gekennzeichnet, dass** die Dicke der Schicht 0,1 bis 10 mm, vorzugsweise 0,5 bis 3 mm, besonders bevorzugt 0,5 bis 0,8 mm beträgt.

10. Verfahren zur Erfassung der biologischen Wirkung von Substanzen (2), wobei
a.) die zu testende Probe (9) auf oder in die Oberfläche eines Trägers (1, 44) gebracht wird, oder bereits Bestandteil einer zu testenden Oberfläche ist,
b.) der Träger (1, 44) mit einer Sensorschicht belegt wird, sofern die Sensorschicht (3) nicht selbst als Träger (1, 44) dient, wobei die Sensorschicht (3) aus einer diffusionskontrollierenden Matrix und darin suspendierten Sensoren (7) besteht,
c.) die Wirkung der in der Probe (9) enthaltenen Substanz (2) oder Substanzen (2) auf die Sensoren (7) in der Sensorschicht (3) bestimmt wird,
**dadurch gekennzeichnet, dass**
in 50 ml Sensorschichtmasse 2 bis 8 ml, bevorzugt 3 bis 5 ml, Reportergenzellsuspension enthalten sind und die Reportergeniellen biolumineszent sind.

11. Verfahren nach Anspruch 10, **dadurch gekennzeichnet, dass** die Sensorschicht (3) selber als Träger (1) verwendet wird.

12. Verfahren nach Anspruch 10 oder 11, **dadurch gekennzeichnet, dass** vor der Bestimmung der Wirkung der in der Probe (9) enthaltenen Substanzen (2) auf die Reportergenzellen ein Inkubationsschritt eingelegt wird, indem die Sensorschicht (3) oder der mit der Sensorschicht (3) belegte Träger (1, 44) entsprechend den Anforderungen der eingesetzten Zellinien unter definierten Bedingungen bezüglich Temperatur, Feuchtigkeit und Begasung eine vorgegebene Zeit gelagert wird.

13. Verfahren nach einem der Ansprüche 10 bis 12, **dadurch gekennzeichnet, dass** die Wirkung der Substanz (2) auf die Reportergenzellen in einer ortsabhängigen Induktion oder Löschung der Lichtemission aus Biolumineszenzprozessen besteht.

14. Verfahren nach einem der Ansprüche 10 bis 13, **dadurch gekennzeichnet, dass** die Substanzen (2) der Probe (9) durch spezifische oder unspezifische Adsorption an geeigneten Trägermaterialien angereichert werden, bevor sie in Kontakt mit der Sensorschicht (3) gebracht werden.

15. Verfahren nach einem der Ansprüche 10 bis 14, **dadurch gekennzeichnet, dass** die zu testende Probe (9) ein Substanzgemisch ist, das chromatographisch oder elektrophoretisch oder mit anderen analytischen oder präparativen Separationstechniken in Fraktionen getrennt wird, bevor es in Kontakt mit der Sensorschicht (3) gebracht wird.

16. Verfahren nach Anspruch 15, **dadurch gekennzeichnet, dass** die chromatographische Trennung in Fraktionen in einer Chomatographiesäule stattfindet und das Eluat fortlaufend entweder kontinuierlich oder in Intervallen auf verschiedene Stellen des Trägers (1, 44) aufgebracht wird (42).

17. Verfahren nach Anspruch 15, **dadurch gekennzeichnet, dass** die chromatographische Trennung in Fraktionen durch Dünnschichtchromatographie oder Elektrophorese auf dem Träger (1, 44) erfolgt, der mit der Sensorschicht (3) belegt wird.

18. Verfahren nach einem der Ansprüche 10 bis 17, **dadurch gekennzeichnet, dass** die zu testende Probe (9) ein Substanzgemisch ist und die Erfassung der biologischen Wirkung der Einzelsubstanzen des Substanzgemisches mit einer Detektion der Struktur der Einzelsubstanzen verknüpft wird, indem das Substanzgemisch chromatographisch oder elektrophoretisch oder mit anderen analytischen oder präparativen Separationstechniken in Fraktionen getrennt und jede Fraktion spektroskopisch untersucht wird, bevor sie in Kontakt mit der Sensorschicht (3) gebracht wird.

19. Verfahren nach Anspruch 18, **dadurch gekennzeichnet, dass** eine Auswertung der Daten der spektroskopischen Untersuchung nur für die Fraktionen erfolgt, an denen durch die Sensorschicht (3) eine biologische Wirkung nachgewiesen werden kann.

20. Verfahren nach Anspruch 18 oder 19, **dadurch gekennzeichnet, dass** die chromatographische Trennung des Substanzgemisches in Fraktionen in einer Chomatographiesäule stattfindet und ein Teil des Eluats fortlaufend auf verschiedene Stellen des Trägers (1, 44) aufgebracht wird, während gleichzeitig ein anderer Teil des Eluats abgezweigt und durch ein Massenspektrometer (47) oder ein NMR-Spektrometer(48) oder ein IR-Spektrometer spektroskopiert wird.

21. Verfahren nach Anspruch 20, **dadurch gekennzeichnet, dass** das Eluat aus der Chromatographiesäule vor Abzweigung des zu spektroskopierenden Anteils durch einen UV-Detektor(46) detektiert wird.

22. Verfahren nach Anspruch 18 oder 19, **dadurch gekennzeichnet, dass** die chromatographische Trennung des Substanzgemisches in einzelne Substanzzonen mittels Dünnschichtchromatographie oder Elektrophorese auf dem Träger (1, 44) erfolgt und von den einzelnen Substanzzonen durch MALDI-Massenspektroskopie oder Raman-Spektroskopie oder andere spektroskopische Verfahren wie UV-VIS oder IR Spektren aufgenommen werden bevor der Träger (1, 44) mit der Sensorschicht (3) belegt wird.

23. Vorrichtung zur Erfassung der biologischen Wirkung von Substanzen (2) bestehend aus einer Sensorschicht (3) gemäß einem der Ansprüche 1 bis 9, die in Kontakt mit der zu testenden Probe (9) steht und einem bildgebenden System, in dessen Detektionsbereich sich ein Teil oder die gesamte Sensorschicht (3) befindet.

24. Vorrichtung nach Anspruch 23, **dadurch gekennzeichnet, dass** die Reportergenzellen der Sensorschicht (3) ihre Aktivität durch Emission oder Löschung von Biolumineszenz anzeigen, und dass das bildgebende System diese Biolumineszenz detektiert.

## Claims

1. Sensor layer (3) for detecting the biological effect of substances (2), where the sensor layer (3) consists of a diffusion-controlling matrix and sensors (7) suspended therein, **characterized in that** 2 to 8 ml, preferably 3 to 5 ml, of reporter gene cell suspension are present in 50 ml of sensor layer composition, and the reporter gene cells are bioluminescent.

2. Sensor layer (3) according to Claim 1, **characterized in that** the matrix is a gel such as agarose, polyacrylates or a viscous solution.

3. Sensor layer (3) according to Claim 1 or 2, **characterized in that** a plurality of different types of reporter gene cells or one type of reporter gene cells able to indicate different biological effects are suspended in the sensor layer (3).

4. Sensor layer (3) according to any of Claims 1 to 3, **characterized in that** the sensor layer (3) contains additions which control or assist the detection process.

5. Sensor layer (3) according to Claim 4, **characterized in that** the additions are buffers for regulating the vitality status of reporter gene cells.

6. Sensor layer (3) according to any of Claims 1 to 5, **characterized in that** the sensor layer (3) contains bioluminescent substrates.

7. Sensor layer (3) according to any of Claims 1 to 6, **characterized in that** the sensor layer (3) consists of a plurality of part-layers, it being possible for the part-layers to differ in thickness and to differ by the type and amount of reporter gene cells and/or additions.

8. Sensor layer (3) according to any of Claims 1 to 7, **characterized in that** the reporter gene cell suspension has an optical density of 0.6 to 1.4 at 660 nm.

9. Sensor layer (3) according to any of Claims 1 to 8, **characterized in that** the thickness of the layer is 0.1 to 10 mm, preferably 0.5 to 3 mm, particularly preferably 0.5 to 0.8 mm.

10. Method for detecting the biological effect of substances (2), in which
a.) the sample (9) to be assayed is put onto or into the surface of a carrier (1,44), or is already a constituent of a surface to be assayed,
b.) the carrier (1,44) is covered with a sensor layer unless the sensor layer (3) itself serves as carrier (1,44), the sensor layer (3) consisting of a diffusion-controlling matrix and sensors (7) suspended therein,
c.) the effect of the substance (2) or substances (2) present in the sample (9) on the sensors (7) in the sensor layer (3) is determined,
**characterized in that** 2 to 8 ml, preferably 3 to 5 ml, of reporter gene cell suspension are present in 50 ml of sensor layer composition, and the reporter gene cells are bio-luminescent.

11. Method according to Claim 10, **characterized in that** the sensor layer (3) is itself used as carrier (1).

12. Method according to Claim 10 or 11, **characterized in that** the determination of the effect of the substances (2) present in the sample (9) on the reporter gene cells is preceded by an incubation step in which the sensor layer (3) or the carrier (1,44) covered with the sensor layer (3) is stored in accordance with the requirements of the cell lines employed under defined conditions in relation to temperature, humidity and gas introduction for a preset time.

13. Method according to any of Claims 10 to 12, **characterized in that** the effect of the substance (2) on the reporter gene cells consists of location-dependent induction or quenching of the emission of light from bioluminescent processes.

14. Method according to any of Claims 10 to 13, **characterized in that** the substances (2) in the sample (9) are concentrated by specific or nonspecific adsorption onto suitable carrier materials before they are brought into contact with the sensor layer (3).

15. Method according to any of Claims 10 to 14, **characterized in that** the sample (9) to be assayed is a mixture of substances which is fractionated by chromatography or electrophoresis or using other analytical or preparative separation techniques before it is brought into contact with the sensor layer (3).

16. Method according to Claim 15, **characterized in that** the chromatographic separation into fractions takes place in a chromatography column, and the eluate is applied (42) either continuously or at intervals to various points on the carrier (1,44).

17. Method according to Claim 15, **characterized in that** the chromatographic separation into fractions takes place by thin-layer chromatography or electrophoresis on the carrier (1,44) which is covered with the sensor layer (3).

18. Method according to any of Claims 10 to 17, **characterized in that** the sample (9) to be assayed is a mixture of substances, and the detection of the biological effect of the individual substances in the mixture of substances is linked to a detection of the structure of the individual substances by the mixture of substances being separated into fractions by chromatography or electrophoresis or with other analytical or preparative separation techniques, and each fraction being investigated by spectroscopy before it is brought into contact with the sensor layer (3).

19. Method according to Claim 18, **characterized in that** the data from the spectroscopic investigation are analysed only for the fractions on which a biological effect can be detected by the sensor layer (3).

20. Method according to Claim 18 or 19, **characterized in that** the chromatographic separation of the mixture of substances into fractions takes place in a chromatography column, and part of the eluate is continuously applied to various points on the carrier (1,44), while another part of the eluate is simultaneously diverted through a mass spectrometer (47) or an NMR spectrometer (48) or an IR spectrometer for spectroscopy.

21. Method according to Claim 20, **characterized in that** the eluate from the chromatography column is detected by a UV detector (46) before diverting the portion for spectroscopy.

22. Method according to Claim 18 or 19, **characterized in that** the chromatographic separation of the mixture of substances into single substance zones takes place by thin-layer chromatography or electrophoresis on the carrier (1,44), and spectra are recorded from the single substance zones by MALDI mass spectroscopy or raman spectroscopy or other spectroscopic methods such as UV-VIS or IR before the carrier (1,44) is covered with the sensor layer (3).

23. Apparatus for detecting the biological effect of substances (2) consisting of a sensor layer (3) according to any of Claims 1 to 9 which is in contact with the sample (9) to be assayed, and of an imaging system in whose detection zone a part or the whole of the sensor layer (3) is located.

24. Apparatus according to Claim 23, **characterized in that** the reporter gene cells in the sensor layer (3) indicate their activity by emission or quenching of bioluminescence, and that the imaging system detects this bioluminescence.

## Revendications

1. Couche (3) à capteurs destinée à la détection de l'action biologique de substances (2), la couche (3) à capteurs étant constituée d'une matrice contrôlant la diffusion contenant des capteurs (7) en suspension, **caractérisée en ce que** 2 à 8 ml, avantageusement 3 à 5 ml de suspension de cellules à gène rapporteur sont contenus dans 50 ml de matière formant la couche à capteurs et les cellules à gène rapporteur sont bioluminescentes.

2. Couche (3) à capteurs suivant la revendication 1, **caractérisée en ce que** la matrice est un gel tel qu'agarose, polyacrylates, ou une solution visqueuse.

3. Couche (3) à capteurs suivant la revendication 1 ou 2, **caractérisée en ce qu'**elle contient en suspension plusieurs sortes différentes de cellules à gène rapporteur ou une seule sorte de cellules à gène rapporteur, qui peut indiquer les diverses actions biologiques.

4. Couche (3) à capteurs suivant l'une des revendications 1 à 3, **caractérisée en ce qu'**elle contient des additifs qui contrôlent ou soutiennent le processus de détection.

5. Couche (3) à capteurs suivant la revendication 4, **caractérisée en ce que** les additifs sont des tampons de régulation de l'état de vitalité des cellules à gène rapporteur.

6. Couche (3) à capteurs suivant l'une des revendications 1 à 5, **caractérisée en ce qu'**elle contient des substrats doués de bioluminescence.

7. Couche (3) à capteurs suivant l'une des revendications 1 à 6, **caractérisée en ce qu'**elle est constituée de plusieurs couches partielles qui peuvent avoir des épaisseurs différentes et qui se distinguent par la nature et la quantité de cellules à gène rapporteur et/ou d'additifs.

8. Couche (3) à capteurs suivant l'une des revendications 1 à 7, **caractérisée en ce que** la suspension de cellules à gène rapporteur a une densité optique, à 660 nm, de 0,6 à 1,4.

9. Couche (3) à capteurs suivant l'une des revendications 1 à 8, **caractérisée en ce que** l'épaisseur de la couche est de 0,1 à 10 mm, avantageusement de 0,5 à 3 mm, notamment de 0,5 à 0,8 mm.

10. Méthode de détection de l'action biologique de substances (2), dans laquelle
a) l'échantillon (9) à tester est disposé sur ou dans la surface d'un support (1, 44) ou est déjà un constituant d'une surface à tester,
b) le support (1, 44) est recouvert d'une couche à capteurs, dans la mesure où la couche (3) à capteurs ne sert pas elle-même de support (1, 44), la couche (3) à capteurs étant constituée d'une matrice de contrôle de diffusion dans laquelle des capteurs (7) sont en suspension,
c) l'action de la substance (2) ou des substances (2) contenues dans l'échantillon (9), sur les capteurs (7) contenus dans la couche (3) à capteurs est déterminée,
**caractérisée en ce que**
2 à 8 ml, avantageusement 3 à 5 ml, de suspension de cellules à gène rapporteur sont contenus dans 50 ml de matière formant la couche à capteurs et les cellules à gène rapporteur sont bioluminescentes.

11. Méthode suivant la revendication 10, **caractérisée en ce que** la couche (3) à capteurs est elle-même utilisée comme support (1).

12. Méthode suivant la revendication 10 ou 11, **caractérisée en ce que** la détermination de l'action des substances (2) contenues dans l'échantillon (9), sur les cellules à gène rapporteur est précédée d'une étape d'incubation dans laquelle la couche (3) à capteurs ou le support (1, 44) recouvert de la couche (3) à capteurs est stocké pendant une période prédéterminée dans des conditions définies de température, d'humidité et de ventilation conformément aux exigences des lignées cellulaires utilisées.

13. Méthode suivant l'une des revendications 10 à 12, **caractérisée en ce que** l'action de la substance (2) sur les cellules à gène rapporteur réside dans une induction ou une extinction à dépendance locale de l'émission de lumière par des processus de bioluminescence.

14. Méthode suivant l'une des revendications 10 à 13, **caractérisée en ce que** les substances (2) de l'échantillon (9) sont enrichies par adsorption spécifique ou non spécifique en matières de support convenables, avant d'entrer en contact avec la couche (3) à capteurs.

15. Méthode suivant l'une des revendications 10 à 14, **caractérisée en ce que** l'échantillon (9) à tester est un mélange de substances qui est séparé en fractions par chromatographie ou par électrophorèse ou par d'autres techniques analytiques ou préparatives de séparation avant d'entrer en contact avec la couche (3) à capteurs.

16. Méthode suivant la revendication 15, **caractérisée en ce que** la séparation chromatographique en fractions a lieu dans une colonne de chromatographie et l'éluat est déposé en permanence sans interruption ou par intervalles en divers endroits du support (1, 44).

17. Méthode suivant la revendication 15, **caractérisée en ce que** la séparation chromatographique en fractions est effectuée par chromatographie sur couche mince ou par électrophorèse sur le support (1, 44) qui est recouvert de la couche (3) à capteurs.

18. Méthode suivant l'une des revendications 10 à 17, **caractérisée en ce que** l'échantillon (9) à tester est un mélange de substances et la détection de l'action biologique des substances individuelles du mélange de ses substances est lié à une détection de la structure des substances individuelles, par le fait que le mélange de substances est séparé en fractions par chromatographie ou par électrophorèse ou par d'autres techniques analytiques ou préparatives de séparation et chaque fraction est examinée par spectroscopie avant d'entrer en contact avec la couche (3) à capteurs.

19. Méthode suivant la revendication 18, **caractérisée en ce qu'**une exploitation des données de l'examen spectroscopique n'est effectuée que pour les fractions pour lesquelles une action biologique peut être détectée par la couche (3) à capteurs.

20. Méthode suivant la revendication 18 ou 19, **caractérisée en ce que** la séparation chromatographique du mélange de substances en fractions a lieu dans une colonne de chromatographie et une partie de l'éluat est continuellement déposée en divers endroits du support (1, 44), tandis qu'une autre partie de l'éluat est en même temps détournée et examinée par spectroscopie à l'aide d'un spectromètre de masse (47) ou d'un spectromètre RMN (48) ou d'un spectromètre IR.

21. Méthode suivant la revendication 20, **caractérisée en ce que** l'éluat sortant de la colonne de chromatographie est détecté par un détecteur UV (46) avant le détournement de la fraction à examiner par spectroscopie.

22. Méthode suivant la revendication 18 ou 19, **caractérisée en ce que** la séparation chromatographique du mélange de substances en zones individuelles de substance est effectuée par chromatographie sur couche mince ou par électrophorèse sur le support (1 ; 44) et des spectres des diverses zones de substances sont enregistrés par spectroscopie de masse MALDI ou spectroscopie Raman ou par d'autres méthodes spectroscopiques telles que UV-VIS ou IR avant que le support (1, 44) soit recouvert de la couche (3) à capteurs.

23. Dispositif pour la détection de l'action biologique de substances (2) constitué d'une couche (3) à capteurs suivant l'une des revendications 1 à 9 qui est au contact de l'échantillon (9) à tester, et d'un système formant une image, dans la zone de détection duquel se trouve une partie ou la totalité de la couche (3) à capteurs.

24. Dispositif suivant la revendication 23, **caractérisé en ce que** les cellules à gène rapporteur de la couche (3) à capteurs manifestent leur activité par émission ou extinction d'une bioluminescence, et **en ce que** le système formant une image détecte cette bioluminescence.
